# EUROPEAN PATENT APPLICATION

(11) **EP 4 700 736 A1**
(43) Date of publication of application: **25.02.2026**
(21) Application number: 25194185.2
(22) Date of filing: 05.08.2025
(51) Int. Cl.: G08B 29/14

(54) **SYNCHRONIZING A FIRE SENSING DEVICE SELF-TEST PROCEDURE**

(30) Priority: 21.08.2024 US 202418810658
(71) Applicant: Honeywell International Inc., Charlotte, NC 28202 (US)
(72) Inventor: WARMERS, Michael, Charlotte, 28202 (US); GASTHUYS, Michael, Charlotte, 28202 (US); BOURAS, Karim, Charlotte, 28202 (US)
(74) Representative: Haseltine Lake Kempner LLP

(57) **Abstract**

Devices, methods, and systems for synchronizing a fire sensing device (200, 300) self-test procedure are described herein. One device includes a memory (124, 524) and a processor (126, 526) to execute instructions stored in the memory to cause a test medium to be generated, initiate a release of the test medium into a testing chamber (104, 204) at a first predetermined time (410), and cause a sensor to take a reading for the test medium at a second predetermined time (412).

## Description

### Technical Field

The present disclosure relates generally to devices, methods, and systems for synchronizing a fire sensing device self-test procedure.

### Background

Large facilities (e.g., buildings), such as commercial facilities, office buildings, hospitals, and the like, may have a fire alarm system that can be triggered during an emergency situation (e.g., a fire) to warn occupants to evacuate. For example, a fire alarm system may include a fire control panel and a plurality of fire sensing devices (e.g., smoke detectors), located throughout the facility (e.g., on different floors and/or in different rooms of the facility) that can sense a fire occurring in the facility and provide a notification of the fire to the occupants of the facility via alarms.

Maintaining the fire alarm system can include regular cleaning and testing of fire sensing devices. Such cleaning and/or testing of fire sensing devices may be mandated by codes of practice in an attempt to ensure that the fire sensing devices are functioning properly.

### Brief Description of the Drawings

Figure 1 illustrates a block diagram of a fire sensing device in accordance with one or more embodiments of the present disclosure.
Figure 2 illustrates a portion of an example of a fire sensing device in accordance with one or more embodiments of the present disclosure.
Figure 3 illustrates a block diagram of a fire alarm system in accordance with one or more embodiments of the present disclosure.
Figure 4 illustrates a timing diagram for synchronizing a fire sensing device self-test procedure in accordance with an embodiment of the present disclosure.
Figure 5 is an example of a controller for synchronizing a fire sensing device self-test procedure, in accordance with one or more embodiments of the present disclosure.

### Detailed Description

Devices, methods, and systems for synchronizing a fire sensing device self-test procedure are described herein. One device includes a memory and a processor to execute instructions stored in the memory to cause a test medium to be generated, initiate a release of the test medium into a testing chamber at a first predetermined time, and cause a sensor to take a reading for the test medium at a second predetermined time.

As mentioned above, maintaining the fire alarm system can include regular cleaning and testing of fire sensing devices. However, since tests may only be completed periodically, there is a risk that faulty fire sensing devices may not be discovered quickly or that tests will not be carried out on all the fire sensing devices in a fire alarm system.

Testing each fire sensing device can be time consuming, expensive, and disruptive to a business. For example, a maintenance engineer is often required to access fire sensing devices which are situated in areas occupied by building users or parts of buildings that are often difficult to access (e.g., elevator shafts, high ceilings, ceiling voids, etc.). As such, the maintenance engineer may take several days and several visits to complete testing of the fires sensing devices, particularly at a large site. Additionally, it is often the case that many fire sensing devices never get tested because of access issues.

In order to ensure fire sensing devices are tested, fire sensing devices can utilize a self-test procedure. The self-test procedure can be an automatic procedure performed by a fire sensing device without a user, such as a maintenance engineer or other type of user. The self-test procedure can therefore allow fire sensing devices to be tested, even if such fire sensing devices are remotely located and/or difficult to access.

The self-test procedure can include generating a test medium and providing the test medium to a test chamber for sensing. The test medium can be provided from a self-test module included in the fire sensing device. However, the self-test module is typically small and can include small variations in the generation of the test medium. Additionally, in some examples the self-test procedure may be affected by the ambient temperature and/or external airflow in the space in which the fire sensing device is located, as well as residue that can build in the testing chamber over the lifetime of the sensing device. The reliability of the self-test may be affected as a result.

Synchronizing a fire sensing device self-test procedure, according to the disclosure, can allow for periodic sensing by a sensor according to a particular timing synchronization. The generated test medium can be periodically released into the testing chamber and the sensor can periodically take a reading at predetermined times. Such a synchronization can provide for a more reliable self-test procedure, even in varying environmental conditions (e.g., external airflow changes, ambient temperature changes, etc.), as compared with previous approaches.

In the following detailed description, reference is made to the accompanying drawings that form a part hereof. The drawings show by way of illustration how one or more embodiments of the disclosure may be practiced.

These embodiments are described in sufficient detail to enable those of ordinary skill in the art to practice one or more embodiments of this disclosure. It is to be understood that other embodiments may be utilized and that mechanical, electrical, and/or process changes may be made without departing from the scope of the present disclosure.

As will be appreciated, elements shown in the various embodiments herein can be added, exchanged, combined, and/or eliminated so as to provide a number of additional embodiments of the present disclosure. The proportion and the relative scale of the elements provided in the figures are intended to illustrate the embodiments of the present disclosure and should not be taken in a limiting sense.

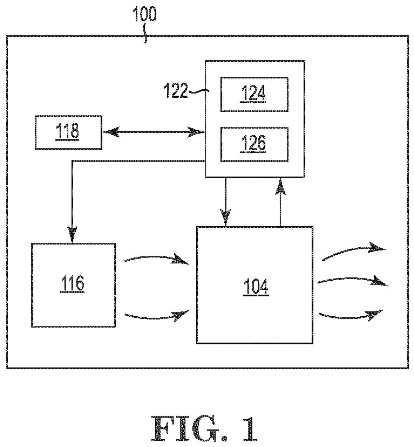

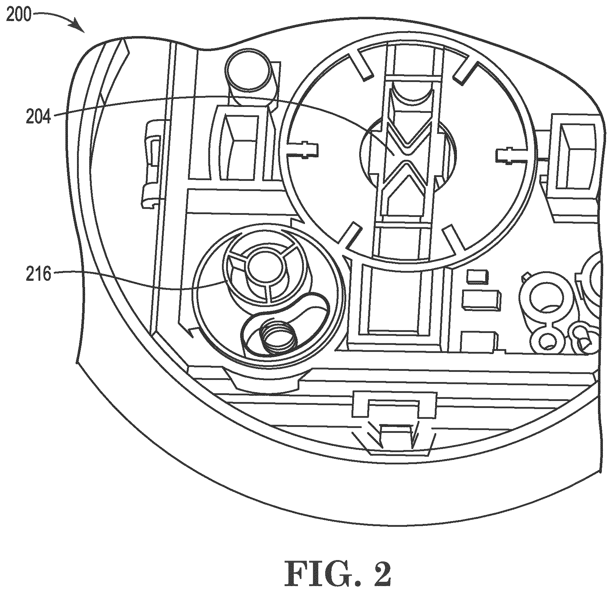

The figures herein follow a numbering convention in which the first digit or digits correspond to the drawing figure number and the remaining digits identify an element or component in the drawing. Similar elements or components between different figures may be identified by the use of similar digits. For example, 104 may reference element "04" in Figure 1, and a similar element may be referenced as 204 in Figure 2.

As used herein, "a", "an", or "a number of" something can refer to one or more such things, while "a plurality of" something can refer to more than one such things. For example, "a number of components" can refer to one or more components, while "a plurality of components" can refer to more than one component.

Figure 1 illustrates a block diagram of a fire sensing device 100 in accordance with one or more embodiments of the present disclosure. The fire sensing device 100 can include a controller (e.g., microcontroller) 122, a sounder 118, an optical scatter chamber 104, and an air movement device 116.

The controller 122 can include a memory 124 and a processor 126. Memory 124 can be any type of storage medium that can be accessed by processor 126 to perform various examples of the present disclosure. For example, memory 124 can be a non-transitory computer readable medium having computer readable instructions (e.g., computer program instructions) stored thereon that are executable by processor 126 to synchronize a fire sensing device self-test procedure in accordance with the present disclosure. For instance, processor 126 can execute the executable instructions stored in memory 124 to cause a test medium to be generated, initiate a release of the test medium into the optical scatter chamber 104 at a first predetermined time, and cause a sensor to take a reading for the test medium at a second predetermined time.

Figure 2 illustrates a portion of an example of a fire sensing device 200 in accordance with one or more embodiments of the present disclosure. The fire sensing device 200 can correspond to the fire sensing device 100 of Figure 1 and can be, but is not limited to, a fire and/or smoke detector of a fire control system.

A fire sensing device 200 can sense a fire occurring in a facility and trigger a fire response to provide a notification of the fire to occupants of the facility. A fire response can include visual and/or audio alarms, for example. A fire response can also notify emergency services (e.g., fire departments, police departments, etc.) In some examples, a plurality of fire sensing devices can be located throughout a facility (e.g., on different floors and/or in different rooms of the facility).

As shown in Figure 2, fire sensing device 200 can include an optical scatter chamber 204 and an air movement device 216, which can correspond to the optical scatter chamber 104 and the air movement device 116 of Figure 1, respectively. Although an air movement device 216 is illustrated in Figure 2, any device capable of initiating a release of a test medium into the optical scatter chamber 204 can be used. For example, a variable airflow generator or a shaker device could be used instead of and/or in combination with air movement device 216.

The air movement device 216 can control the airflow through the fire sensing device 200, including the optical scatter chamber 204. For example, the air movement device 216 can move particles, gases, and/or aerosol from a first end of the fire sensing device 200 to a second end of the fire sensing device 200. The air movement device 216 can start responsive to a command and can stop responsive to a command and/or after a particular period of time, as is further described herein.

A fire sensing device 200 can automatically or upon command perform a self-test procedure. The self-test procedure can include causing a test medium to be generated, initiating a release of the test medium into the optical scatter chamber 204, and causing a sensor to take a reading for the test medium. The controller can initiate the release of the test medium at a first predetermined time and cause the sensor to take a reading for the test medium at a second predetermined time, as is further described herein.

The reading can include measuring a value associated with the test medium in the optical scatter chamber 204. The controller can further compare the value associated with the test medium to a threshold value.

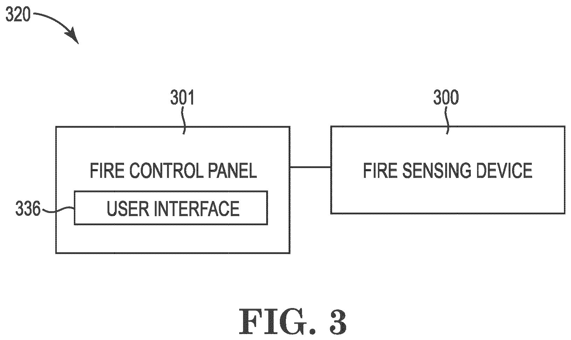

Figure 3 illustrates a block diagram of a fire alarm system 320 in accordance with one or more embodiments of the present disclosure. The fire alarm system 320 can include a fire sensing device 300 and a fire control panel 301. Fire sensing device 300 can be, for example, fire sensing device 100 and/or 200 previously described in connection with Figures 1 and 2, respectively.

The fire control panel 301 can be a monitoring device, a fire detection control system, and/or a cloud computing device of the fire alarm system 320. The fire control panel 301 can be configured to send commands to and/or receive reports from a fire sensing device 300 via a wired or wireless network. For example, the fire sensing device 300 can report a sensor reading during a self-test procedure of the fire sensing device 300. Additionally, in some examples the fire sensing device 300 can report a confirmed fire to the fire control panel 301 responsive to a measured value after a particular period of time being greater than a threshold value.

The fire control panel 301 can receive reports from a number of fire sensing devices analogous to fire sensing device 300. For example, the fire control panel 301 can receive reports from each of a number of fire sensing devices analogous to fire sensing device 300 and transmit commands based on the reports from each of the number of fire sensing devices.

In a number of embodiments, the fire control panel 301 can include a user interface 336. The user interface 336 can be a GUI that can provide and/or receive information to and/or from a user and/or the fire sensing device 300. The user interface 336 can display messages and/or data received from the fire sensing device 300. For example, the user interface 336 can alert a user to an unconfirmed fire, a confirmed fire, and/or a false alarm reported by the fire sensing device 300.

The networks described herein can be a network relationship through which fire sensing device 300 and/or fire control panel 301 can communicate with each other. Examples of such a network relationship can include a distributed computing environment (e.g., a cloud computing environment), a wide area network (WAN) such as the Internet, a local area network (LAN), a personal area network (PAN), a campus area network (CAN), or metropolitan area network (MAN), among other types of network relationships. For instance, the network can include a number of servers that receive information from and transmit information to fire sensing device 300 and/or fire control panel 301 via a wired or wireless network.

As used herein, a "network" can provide a communication system that directly or indirectly links two or more computers and/or peripheral devices and allows a fire control panel to access data and/or resources on a fire sensing device 300 and vice versa. A network can allow users to share resources on their own systems with other network users and to access information on centrally located systems or on systems that are located at remote locations. For example, a network can tie a number of computing devices together to form a distributed control network (e.g., cloud).

A network may provide connections to the Internet and/or to the networks of other entities (e.g., organizations, institutions, etc.). Users may interact with network-enabled software applications to make a network request, such as to get data. Applications may also communicate with network management software, which can interact with network hardware to transmit information between devices on the network.

In some examples, the network can be used by the fire sensing device 300 and/or the fire control panel 301 to communicate with a computing device. The computing device can be a personal laptop computer, a desktop computer, a mobile device such as a smart phone, a tablet, a wrist-worn device, and/or redundant combinations thereof, among other types of computing devices. The computing device can receive reports from a number of fire sensing devices analogous to fire sensing device 300 and/or a number of fire control panels analogous to fire control panel 301 and transmit commands based on the reports to one or more of the number of fire sensing devices and/or one or more of the number of fire control panels.

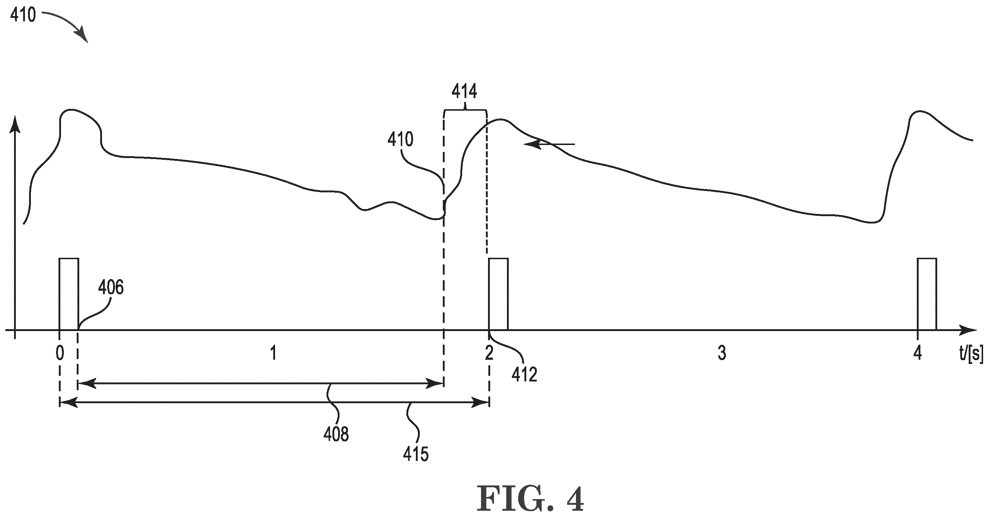

Figure 4 illustrates a timing diagram for synchronizing a fire sensing device self-test procedure in accordance with an embodiment of the present disclosure. In some embodiments, the steps of the timing diagram can be performed by a controller of the fire sensing device, previously described in connection with Figures 1, 2, and/or 3.

As mentioned above, a controller of a fire sensing device can cause a self-test procedure to occur. The self-test procedure can be a test to ensure the fire sensing device accurately detects smoke particulates in the area in which the fire sensing device is located. The self-test procedure can be synchronized between the generation of the testing medium and the sensing of the test medium, as is further described herein.

The timing diagram of Figure 4 illustrates an amount of test medium inside of a testing chamber (e.g., an optical scatter chamber) of the fire sensing device. For example, at 406, the controller can cause a test medium to be generated. In some examples, the test medium can be an aerosol. For example, the controller can cause a coil to heat wax until a temperature at which the wax emits an aerosol comprised of smoke particles. The coil and the wax can be located in a self-test module of the fire sensing device, as mentioned above.

While the test medium is described above as being an aerosol, embodiments of the present disclosure are not so limited. For example, the test medium can be, for example, a gas, light, etc.

The controller can initiate the self-test procedure when the fire sensing device is in a test mode. When the fire sensing device is in the test mode, particles sensed in the testing chamber that exceed a threshold amount can be detected, but will not initiate an alarm in the facility.

Test medium can be generated over a test medium aggregation period 408. For example, the self-test module can generate the test medium over a period of time indicated by the test medium aggregation period 408. During the test medium aggregation period 408, the test medium is not provided to the testing chamber (e.g., the optical scatter chamber). Accordingly, as illustrated in Figure 4, the concentration of test medium in the testing chamber declines.

The controller can initiate a release of the test medium into the testing chamber (e.g., the optical scatter chamber) at the first predetermined time 410. As mentioned above, the testing chamber can be an optical scatter chamber to measure a value associated with the test medium located therein. The controller can initiate the release of the test medium into the optical scatter chamber during the self-test procedure as is further described herein.

In order to ensure there is a sufficient amount of test medium in the optical scatter chamber during the sensing, the release of the test medium and the sensing can be performed at specific times. This synchronization can allow for a more reliable self-test, as compared with previous approaches.

For example, as mentioned above, the controller can initiate the release of the test medium into the testing chamber at the first predetermined time 410. The first predetermined time 410 can be after the generation of the test medium during the test medium aggregation period 408. In other words, the first predetermined time 410 can occur a predefined period of time (e.g., the test medium aggregation period) after initiation of the generation of the test medium.

In order to initiate the release of the test medium into the optical scatter chamber, the controller can cause the air movement device to turn on for a predetermined runtime period 414. For example, as illustrated in Figure 4, after the first predetermined time 410, the controller can cause the air movement device to turn on for the predetermined runtime period 414. In an example in which the test medium is an aerosol, the air movement device can be turned on for the duration of the predetermined runtime period 414 to cause the aerosol generated during the test medium aggregation period 408 to be released into the testing chamber (e.g., the optical scatter chamber).

Although the test medium is described above as being an aerosol, embodiments of the present disclosure are not so limited. For instance, in another example, the test medium can be a gas, and the controller can cause the gas generated during the test medium aggregation period 408 to be released into the testing chamber at the first predetermined time 410. As another example, the test medium can be light, and the controller can cause the light to be released into the testing chamber at the first predetermined time 410.

As illustrated in Figure 4, the test medium aggregation period 408 occurs before the first predetermined time 410, and after the first predetermined time 410, the controller initiates the release of the test medium into the optical scatter chamber during the predetermined runtime period 414. The predetermined runtime period 414 of the air movement device occurs before the second predetermined time 412, as is further described herein.

The predetermined runtime period 414 can be a period of time to enable a sufficient amount of test medium to be moved into the optical scatter chamber. After the predetermined runtime period 414, the controller can cause the air movement device to turn off.

The controller can cause a sensor in the optical scatter chamber to take a reading for the test medium at the second predetermined time 412. The second predetermined time 412 can be a predefined period of time after the first predetermined time 410. For example, the period of time between the first predetermined time 410 and the second predetermined time 412 can be two seconds and can allow for a sufficient amount of test medium to be moved into the optical scatter chamber (e.g., by the air movement device when the air movement device is turned on).

Although the controller is described above as causing the sensor to take the reading for the test medium at the second predetermined time 412, embodiments are not so limited. For example, the first predetermined time 410 can be a particular amount of time before the second predetermined time 412. The second predetermined time 412 can be a point at which the sensor is preprogrammed to take a reading of the value associated with the medium in the optical scatter chamber. The first predetermined time 410 and the second predetermined time 412 can be selected to allow for a predetermined period of time to elapse between the first predetermined time 410 and the second predetermined time 412 such that a sufficient amount of test medium can be moved into the optical scatter chamber and the sensor can take the reading of the value associated with the medium in the optical scatter chamber.

Although the period of time between the first predetermined time 410 and the second predetermined time 412 is described above as being two seconds, embodiments of the present disclosure are not so limited. For example, the period of time between the first predetermined time 410 and the second predetermined time 412 can be variable. For instance, the period of time between the first predetermined time 410 and the second predetermined time 412 can be less than two seconds or more than two seconds.

At the second predetermined time 412, the sensor can take a reading. The sensor can include a measurement of a value associated with the test medium in the optical scatter chamber. The value can be, for example, an obscuration and/or scattering value. The sensor can take a reading, for instance, for two milliseconds, although embodiments of the disclosure are not limited to a sensor reading of two milliseconds.

In some examples, the controller can compare the measured value to a threshold value. For example, the controller can determine the measured quantity of particles to be a particular measured scatter value, and can compare the measured scatter value to a threshold scatter value.

Based on the comparison, the controller can determine whether the self-test procedure was successful. For example, if the measured scatter value exceeds the threshold scatter value, the controller can determine the self-test procedure was successful. If the measured scatter value does not exceed the threshold scatter value, the controller can determine the self-test procedure was not successful.

In the example in which the controller compares the measured scatter value to the threshold scatter value and determines based on the comparison whether the self-test procedure was successful, the controller can transmit whether the self-test procedure was successful to a computing device and/or to a fire control panel. The results of the self-test procedure can be utilized by a user to determine whether the fire sensing device should be cleaned, serviced, replaced, etc.

In some examples, the controller can determine a scatter value. In such an example, the controller can transmit the reading of the scatter value in the optical scatter chamber to a computing device and/or a fire control panel. The measured scatter value may be utilized by the computing device, the fire control panel, and/or manually by a user to determine whether the self-test procedure was successful, and correspondingly, whether the fire sensing device should be cleaned, serviced, replaced, etc.

As illustrated in Figure 4, the method can be repeated at a particular predetermined frequency 415. For example, as described above, the controller can cause the test medium to be generated, initiate the release of the test medium into the testing chamber at the first predetermined time, and cause the sensor to take the reading for the test medium at the second predetermined time every two seconds. The method can be repeated at the particular predetermined frequency 415 for the duration of the test mode of the fire sensing device, or for less than the duration of the test mode of the fire sensing device.

Although the particular predetermined frequency 415 is described above as being two seconds, embodiments of the present disclosure are not so limited. For example, the particular predetermined frequency 415 can be more than two seconds or less than two seconds. Additionally, the test medium aggregation period 408, the first predetermined time 410, the predetermined runtime period 414, and/or the second predetermined time 412 may be modified to ensure the synchronization between the test medium generation, release of the test medium into the optical scatter chamber, and the sensor reading, as described above.

Accordingly, synchronizing a fire sensing device self-test procedure according to the disclosure can allow for periodic sensing by the sensor according to a particular timing synchronization. The synchronization can ensure that a sufficient amount of test medium is provided to the testing chamber and that the sensor takes a sensor reading while the sufficient amount of test medium is in the testing chamber to provide for a more reliable self-test procedure, even in varying environmental conditions (e.g., external airflow changes, ambient temperature changes, etc.), as compared with previous approaches.

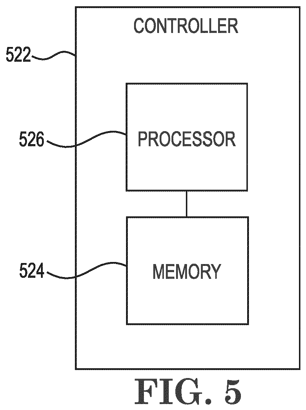

Figure 5 is an example of a controller 522 for synchronizing a fire sensing device self-test procedure, in accordance with one or more embodiments of the present disclosure. As illustrated in Figure 5, the controller 522 can include a memory 524 and a processor 526 for synchronizing a fire sensing device self-test procedure, in accordance with the present disclosure.

The memory 524 can be any type of storage medium that can be accessed by the processor 526 to perform various examples of the present disclosure. For example, the memory 524 can be a non-transitory computer readable medium having computer readable instructions (e.g., executable instructions/computer program instructions) stored thereon that are executable by the processor 526 for synchronizing a fire sensing device self-test procedure in accordance with the present disclosure.

The memory 524 can be volatile or nonvolatile memory. The memory 524 can also be removable (e.g., portable) memory, or non-removable (e.g., internal) memory. For example, the memory 524 can be random access memory (RAM) (e.g., dynamic random access memory (DRAM) and/or phase change random access memory (PCRAM)), read-only memory (ROM) (e.g., electrically erasable programmable read-only memory (EEPROM) and/or compact-disc read-only memory (CD-ROM)), flash memory, a laser disc, a digital versatile disc (DVD) or other optical storage, and/or a magnetic medium such as magnetic cassettes, tapes, or disks, among other types of memory.

Further, although memory 524 is illustrated as being located within controller 522, embodiments of the present disclosure are not so limited. For example, memory 524 can also be located internal to another computing resource (e.g., enabling computer readable instructions to be downloaded over the Internet or another wired or wireless connection).

The processor 526 may be a central processing unit (CPU), a semiconductor-based microprocessor, and/or other hardware devices suitable for retrieval and execution of machine-readable instructions stored in the memory 524.

Although specific embodiments have been illustrated and described herein, those of ordinary skill in the art will appreciate that any arrangement calculated to achieve the same techniques can be substituted for the specific embodiments shown. This disclosure is intended to cover any and all adaptations or variations of various embodiments of the disclosure.

It is to be understood that the above description has been made in an illustrative fashion, and not a restrictive one. Combination of the above embodiments, and other embodiments not specifically described herein will be apparent to those of skill in the art upon reviewing the above description.

The scope of the various embodiments of the disclosure includes any other applications in which the above structures and methods are used. Therefore, the scope of various embodiments of the disclosure should be determined with reference to the appended claims, along with the full range of equivalents to which such claims are entitled.

In the foregoing Detailed Description, various features are grouped together in example embodiments illustrated in the figures for the purpose of streamlining the disclosure. This method of disclosure is not to be interpreted as reflecting an intention that the embodiments of the disclosure require more features than are expressly recited in each claim.

Rather, as the following claims reflect, inventive subject matter lies in less than all features of a single disclosed embodiment. Thus, the following claims are hereby incorporated into the Detailed Description, with each claim standing on its own as a separate embodiment.

## Claims

1. A controller (122, 522) for a fire sensing device (200, 300), comprising:
a memory (124, 524); and
a processor (126, 526) configured to execute executable instructions stored in the memory (124, 524) to:
cause a test medium to be generated;
initiate a release of the test medium into a testing chamber (104, 204) at a first predetermined time; and
cause a sensor to take a reading for the test medium at a second predetermined time.

2. The controller (122, 522) of claim 1, wherein the first predetermined time is a predefined period of time after initiation of the generation of the test medium.

3. The controller (122, 522) of claim 1, wherein the second predetermined time is a predefined period of time after the first predetermined time.

4. The controller (122, 522) of claim 1, wherein the test medium is an aerosol.

5. The controller (122, 522) of claim 4, wherein the processor (126, 526) is configured to execute the instructions to initiate the release of the aerosol into the testing chamber (104, 204).

6. The controller (122, 522) of claim 1, wherein the test medium is a gas.

7. The controller (122, 522) of claim 6, wherein the processor (126, 526) is configured to execute the instructions to initiate the release of the gas into the testing chamber (104, 204).

8. The controller (122, 522) of claim 1, wherein the test medium is light.

9. The controller (122, 522) of claim 8, wherein the processor (126, 526) is configured to execute the instructions to initiate the release of the light into the testing chamber (104, 204).

10. A fire sensing device (200, 300), comprising:
an air movement device (116, 216); and
a controller (122, 522) configured to:
cause a test medium to be generated for a self-test procedure;
cause, at a first predetermined time, the air movement device (116, 216) to initiate release of the test medium into a testing chamber (104, 204), wherein the first predetermined time is after the generation of the test medium; and
cause, at a second predetermined time, a sensor to take a reading for the test medium, wherein the second predetermined time is after the first predetermined time.

11. The fire sensing device (200, 300) of claim 10, wherein the controller (122, 522) is configured to cause the air movement device (116, 216) to turn on for a predetermined runtime period.

12. The fire sensing device (200, 300) of claim 11, wherein the controller (122, 522) is configured to cause the air movement device (116, 216) to turn off after the predetermined runtime period.

13. The fire sensing device (200, 300) of claim 11, wherein the predetermined runtime period occurs before the second predetermined time.

14. The fire sensing device (200, 300) of claim 10, wherein the testing chamber (104, 204) is an optical scatter chamber (104, 204) configured to:
measure a value associated with the test medium located therein;
compare the measured value to a threshold value; and
determine whether the self-test procedure was successful based on the comparison.

15. The fire sensing device (200, 300) of claim 14, wherein the controller (122, 522) is configured to transmit at least one of the measured value and whether the self-test procedure was successful to a computing device or a fire control panel (301).
